# EUROPEAN PATENT SPECIFICATION

(11) **EP 4 231 992 B1**
(45) Date of publication and mention of the grant of the patent: **04.03.2026**
(21) Application number: 21883570.0
(22) Date of filing: 13.10.2021
(51) Int. Cl.: A61K 8/34, A61K 8/37, A61K 8/60, A61Q 13/00

(54) **ANHYDROUS ALCOHOL-FREE SILKY FRAGRANCE FORMULATION**
WASSERFREIE ALKOHOLFREIE SEIDENDUFTSTOFFFORMULIERUNG
FORMULATION ANHYDRE DE PARFUM SOYEUX SANS ALCOOL

(30) Priority: 21.10.2020 US 202063094581 P
(43) Date of publication of application: 30.08.2023
(73) Proprietor: AKI, Inc., Chattanooga, TN 37406 (US)
(72) Inventor: MEEHAN, Matthew, Teaneck, NJ 07666 (US); O'HALLORAN, David, Somerset, NJ 08873 (US)
(74) Representative: Grund, Martin
(86) International application number: PCT/US2021/054673
(87) International publication number: WO 2022/086766

(56) References cited:
- WO-A1-2014/139963
- WO-A1-2019/154892
- US-A1- 2006 110 415
- US-A1- 2007 190 004
- US-A1- 2015 202 139
- US-A1- 2018 036 210
- US-A1- 2018 140 540
- US-A1- 2021 106 966
- US-B2- 8 227 426

## Description

### FIELD OF THE DISCLOSURE

The disclosed subject matter relates to an anhydrous alcohol-free fragrance carrier formulation for a perfume or cologne end-product that is aesthetically and fragrantly pleasing to consumers. The formulation can be used in silky and/or moisturizing perfume-based translucent product(s). Such formulation comprises perfume compounds, triethyl citrate and ethyl hexyl glycerin, and is characterized by more pronounced intensity, longevity, and character of original fragrance notes, having a luxurious silky feel coupled with a smoothing and/or moisturizing effect when applied to the skin.

### BACKGROUND

Alcohols such as ethanol are commonly used as solvents in the cosmetics industry, especially for fragrances such as perfumes, colognes, aftershaves, and other perfuming substances. Alcohol, however, has known deleterious effects, such as drying and irritation of the skin, skin diseases, and low lasting of the fragrance due to the rapid evaporation of fragrance molecules caused by evaporation of the ethanol, in particular the top notes of the fragrance, which can rapidly alter the overall balance of the originally intended total fragrance. Thus, there is a need for alcohol-free cosmetic and fragrance formulations that avoid the negative effects of ethanol while maintaining the benefits of original fragrance lift from the skin. Furthermore, many consumers enjoy cosmetic products and/or fragrances with a pleasant, silky texture and good clarity. The overall aesthetic quality and luxury feel thereto of such products is often desired by consumers without the sticky and/or tacky feel of such water and alcohol containing fragrance products.

More particularly, ethanol has generally been widely used in the industry as a carrier for fine fragrances. The safety of topical applications of ethanol is still a matter of debate, and there appears to be scientific evidence pointing in both directions. On the one hand, researchers concluded that the range of damage caused to the skin by the alcohol cannot and should not be ignored, although the deleterious effects of ethanol exposure on the skin may pale into insignificance compared to its effects on the liver, central nervous system, and other body systems after ingestion. On the other hand, scientific studies attributed ethanol for topical uses as safe *per se.* However, there appears to be at least some evidence, including epidemiological data, about mouthwash use, and data from animal experiments showing that ethanol on the skin or inside the oral cavity may cause harm if used chronically. Evaluation according to EU cosmetics legislation and other acts about chemical safety should consider the chronic toxic and carcinogenic potential of ethanol. In Lachenmeier's article (Dirk W. Lachenmeier, Safety evaluation of topical applications of ethanol on the skin and inside the oral cavity, J. Occup. Med. Toxicol., 2008; 3: 26), the safety of topical uses of ethanol was evaluated by a critical review of the scientific literature. The author mentioned that ethanol abuse has been associated with the development of several skin disorders such as psoriasis, discoid eczema, and superficial infections. Chronic alcohol abuse was also a predisposing factor for necrotizing wound infections, delayed wound healing and cellulitis, among others.

Traditional perfume is a solution of fragrance compounds (e.g., fragrant essential oils, absolutes, aroma compounds, fixatives) dissolved in selected solvents. The most typical solvent used in perfumery products is ethanol or a mix of water and ethanol (aqueous alcoholic solution). Depending on the concentration of aromatic compounds in the solution the following varieties of fragrance can be distinguished: extrait de parfum, known as parfum (up to 40% fragrance composition, in 90-96% alcoholic solution), eau de parfum, EDP (10-15% fragrance composition in 80-90% aqueous alcoholic solution), eau de toilette, EDT (5-10% fragrance composition in 60-85% aqueous alcoholic solution), aftershave (5% fragrance composition in diluted alcoholic solution), eau de Cologne, commonly called cologne (3-8% aromatic compounds in 70-80% aqueous alcoholic solution) and au fraiche also known as body splash, body spray, body mist or body spritz (up to 3% fragrance composition diluted mainly with water). In these formulations, ethanol is used as a solvent of fragrances but additionally, it serves as an antimicrobial agent. A further advantage of alcohol is its fast-drying rate. On the other hand, it can dry out the skin and cause cutaneous intolerance or allergic contact dermatitis and as consequence, skin irritation and inflammation, among other ill effects, as described in the foregoing.

Alcohol-based perfume is the most common type of fragrance products available on the market. However, due to the potential irritating properties of ethanol, its flammability and even ethical restrictions associated with its use in the Middle East, there is a growing market of alcohol-free perfume products, mainly in the form of solid or liquid oils. Certain religions, forbid the use of any alcohol, where it is considered unlawful for anyone to produce, import, sell and consume alcohol for example, in certain Middle East countries. Hence, there is a need for a globally acceptable formula for any such countries and/or religions that prohibit the use of alcohol in any form.

However, products in alternate forms have a number of drawbacks discussed above. Therefore, new solutions are still being sought with regard to the physical and chemical form of perfume-based products. Even further, there exists a need to expand the market to new consumer groups like children, adolescents, and allergy sufferers, therefore, the elimination of the most commonly used solvent -ethanol- becomes a necessity but it also remains a great challenge.

Accordingly, pressure is now being felt by the cologne and perfume industry to reduce the quantity of alcohol in the products. It would be recommended to make a water-based perfume if such a perfume could have the look and feel of an alcohol-based perfume. However, fragrance ingredients are mostly oils not soluble in water, which could impact the aesthetic factor of such end products. Moreover, it is a challenge to achieve an alcohol-free formulation acceptable to the consumer accustomed to the physical properties of alcohol-based perfume compositions, in particular the clarity and transparency, the clean feeling on the skin and the rapid evaporation rates leading to the non-staining properties on skin and clothing, all considered desirable.

There are some examples of alcohol-free, water-based products described in literature. However, replacing ethyl alcohol completely with water is proven to be a challenging task for scientists. The essences used in perfumery products are inherently mixtures of hydrophobic (water-insoluble) compounds. Therefore, obtaining stable solutions based on water has proven to be challenging. One of the solutions facilitating the introduction of hydrophobic fragrances into water is the use of solubilizers (substances that increase solubility), e.g., glycols, glycerin or surfactants (compounds that lower the surface tension between two liquids), in order to obtain stable dispersions of hydrophobic fragrances in water, in the form of for example, emulsions, microemulsions, micelles, and/or liposomes.

Perfumes are formulations wherein hundreds of discrete organic, hydrophobic fragrance ingredients are blended to produce a final scent. Any attempt to replace part or all of the alcohol in perfume or cologne formulations with non-volatile organic compounds (VOCs) changes the solubility and physico-chemical environment of the aromatic compounds and requires a re-formulation of the diluent and emulsifier in an attempt to achieve final scent, lasting fragrance notes, and tactile properties similar to alcohol containing perfumes or colognes. Reduced solubility can even result in the fragrance compounds precipitating out the solution. Enhancing solubilization by utilization of common surfactants and emulsifiers results in a tacky, soapy, impure feeling on the skin, which is not attractive to consumers. That is, surfactants are surface active agents, compounds which generally have a hydrophobic and a hydrophilic component and are added only for their functionality in bridging the gap between the hydrophobic and hydrophilic phases and improving solubility. While surfactants are necessary to facilitate dissolution or dispersion of fragrance oils in water, they otherwise detract from the final composition. Hence, it is preferred to utilize as little surfactant as possible in such formulations.

Alcohol used in perfume is one of the VOCs which is more and subjected to more regulation. One approach to providing a perfume low in VOCs has been to provide a fragrance delivering compositions as a dry powder. However, while this approach solves the problem of VOCs, the physical properties of the product are so different from traditional perfume compositions that it is not considered as providing an acceptable substitute for perfumes. Another approach developed to this end is to make a non-alcoholic formulation of fragrance.

In WO 2019/154892 A1 and US 2006/110415 A1 alcohol-free perfume compositions are disclosed.

In addition, for example, U.S. Patent No. 5,468,725 describes an alcohol-free transparent perfume containing an alcohol-free perfume base, water, and a stable transparent oil-in-water microemulsion fragrance concentrate. The oil-in-water microemulsion fragrance concentrate contains water, at least one hydrophobic fragrance oil, at least one cationic surfactant and at least one non-ionic surfactant. A cationic surfactant and a non-ionic surfactant are used with an aqueous media and a fragrance oil to prepare a transparent alcohol-free fragrance microemulsion.

Yet another, U.S. Patent No. 6,774,101 relates to an alcohol-free translucent perfuming composition in the form of an oil-in-water emulsion containing at least a perfume oil, a surfactant system having a hydrophilic-lipophilic ratio not less than 10 and water. The use of a high amount of water in the formulations can affect the stability and the rendition of the fragrance. Moreover, the use of surfactants can impact the feeling of freshness and cleanliness when the formulation is applied on the skin in addition to altering fragrance notes of the perfumed composition.

One of the alternatives to alcoholic solutions are oil-based perfumes. Generally for production thereof, virtually any neutral oil can be combined with even the least intense fragrance. Oil as a carrier of aromatic substances cannot mask or distort the scent of the designed fragrance composition. The most commonly used oils are almond oil, jojoba oil or fractionated coconut oil. The essential advantage of oil-based perfumes is that they do not dry out the skin like alcohol solutions. In fact, quite the opposite, oil-based products have been found to moisturize the skin and improve the fragrance longevity. This type of product is commonly known as "skin scents", and the body temperature allows the scent to be released gradually. It is estimated that the scent of oil-based perfumes lasts for 6-15 h, while those based on alcohol last for about 3 h. The fragrance concentration in oil products is quite high, usually around 20%. However, one of the disadvantages of these products is their lack of fresh, lighter notes called "top notes", making the overall smell heavy. Additionally, dispersing these products on the clothes and in the air causes greasy stains and can make surfaces slippery and sticky.

The disadvantages of microemulsions are the high surfactants concentration in the composition (up to 40%). It could be a reason of high products viscosity and/or lack of its transparency. Moreover, fragrances formulated in such compositions can react with surfactant and may undergo degradation processes, which adversely affects the stability and organoleptic properties of the product. In addition, it should be noted that the cationic surfactants described in some of the above patents are known for their irritating properties and therefore should be avoided in 'leave-on products' such as perfume goods. Additionally, these products may require the use of special packaging that is resistant to this type of formulation.

Hence, while there are known fragrance products containing alcohol and high amounts of water, there remains a need for an anhydrous silky cosmetic and/or fragrance carrier formulation that has a silky feel when applied to the skin. The present invention achieves an alcohol-free fragrance formulation without any surfactant or water. The present invention further achieves duration of the original fragrance notes, without altering the originally intended fragrance, yet achieves a translucent, non-sticky formulation.

Yet further, there is a need for a silicone-free fragrance carrier formulation with same above-described benefits. Silicone is a synthetic material which many customers try to avoid, yet it is not completely banned. In addition, to causing eventual dryness to the skin, though it creates an illusion of silk-soft skin, there are also concerns about how silicone affects that environment and some non-biodegradable versions have even been banned by the EU. Silicone has been also found to be difficult to remove from the skin, even leading to acne and other skin irritations, among other potential health concerns, while generally having no tangible long-term benefits to skin.

The present invention solves the different problems described in the foregoing, by means of an anhydrous alcohol-free, silicone-free, and preservative-free fragrance formulation that exhibits good clarity, solubility, and a pleasant smooth feel with non-tacky rub out when applied to the skin. The fragrance carrier composition is composed of a combination of triethyl citrate and ethyl hexyl glycerin.

### SUMMARY

The purpose and advantages of the disclosed subject matter are set forth in the following description.

The invention is defined by the appended claims. Thus, the invention provides an anhydrous alcohol-free fragrance carrier composition comprising at least one perfume compound, present in an amount of about 1 to 40 percent by weight relative to a total weight of the composition; triethyl citrate, present in an amount of about 10 to 50 percent by weight relative to the total weight of the composition; and ethyl hexyl glycerin in an amount of about 10 to 40 percent by weight relative to the total weight of the composition. Such composition is capable of retaining high levels of fragrance notes up to 30% without affecting the character or aesthetics of the composition.

In accordance with a preferred embodiment of the disclosed subject matter, the anhydrous alcohol-free silky fragrance carrier formulation is more preferably composed of: 1 to 40 wt. % of perfume compound(s), preferably 10 to 30 wt. %; 10 to 50 wt. % of triethyl citrate, preferably 10 to 35 wt. %; 5 to 40 wt. % of ethyl hexyl glycerin, preferably 5 to 25 wt. %; and optionally some diluent(s) such as propylene glycol, 1,3 propanediol, dipropylene glycol, isopropyl myristate, isopropyl palmitate or a combination may be added. Natural oils with skin soothing, moisturizing action may also be added of up to 5 wt. %. The silky fragrance carrier formulation is yet further silicone-free and preservative free. The disclosed silky fragrance carrier formulation is characterized by more pronounced intensity, longevity and character of original fragrance notes, with a silky luxurious feel coupled with a smoothing and/or moisturizing effect when applied to the skin. Additional embodiments of the fragrance carrier formulation are disclosed hereinbelow, including one or more preferred embodiments thereof.

### DETAILED DESCRIPTION

The present invention will be described below in more detail by way of specific working examples. Other combinations and various modifications within the conception of the present invention are possible without departing from the subject matter and scope of the present invention.

Unless stated otherwise, percentages (%) are meant to designate percent by weight of a composition.

Herein, all amounts, percentages and ratios are by weight unless otherwise indicated. In addition, al percentages are by weight of the total composition. All amounts, percentages, and ratios are to be understood as prefixed by the word "about".

Herein, the word "comprising" and "comprised of", etc., should be understood as meaning that other components/features could also be present; i.e., the listed steps or options need not be exhaustive.

The composition of the present invention may be applied to the human body by any means. Application of liquid compositions may be by absorption onto a carrier matrix for example, like, paper, fabric, sponge, or otherwise using another means, for example, fragrance oil dropper/applicator, liquid pump. Application is effected by contacting said carrier matrix with the surface of the body or skin. Alternatively, one applies the fragrance composition using for example, an oil dropper/applicator or liquid pump. Application of such fragrance composition may also comprise a combination of any two or more of the above techniques.

Herein, any material, ratio or weigh indicated as "preferred" is to be understood as preferably used in combination within any other material, ratio or weigh indicated as "preferred".

Reference within the specification to "embodiment(s)" or the like means that a particular material, feature, structure and/or characteristic described in connection with the embodiment is included in at least one embodiment, but it does not mean that all embodiments incorporate the material, feature, structure, and/or characteristic described. Furthermore, materials, features, structures and/or characteristics may be combined in any suitable manner across different embodiments and materials, features, structures and/or characteristics may be omitted or substituted from what is described.

"Free of" or stated ingredient "-free" means that the stated ingredient has not been added to the composition. However, the stated ingredient may incidentally form as a byproduct or a reaction product of the other components of the fragrance carrier composition.

By "stable" is meant a composition that does not degrade over time and whose compounds will not react with each other. By "stable" is meant that the homogeneity, transparency, pleasant smell and viscosity of the composition are retained. More particularly, the composition according to the present invention is stable in accelerated aging after 24 hours at UV (Suntest) and after 2 months at 25 ° C. and 45 ° C. in comparison with a sample kept at 5 ° C., protected from light.

By having a "viscosity" close to that of a "slightly viscous oil" is meant that the viscosity of said composition is preferably between 20 and 55 mPa·S (20-55 centipoise).

The present disclosure is directed to a composition for an anhydrous alcohol-free fragrance carrier, for a perfume or cologne-based end product, including other perfuming substances, and further characterized by a luxurious silky formula that melts into your skin while leaving behind a heightened, long-lasting fragrance. In particular, the anhydrous alcohol-free fragrance carrier for a perfume or cologne-based end product encompasses a luxuriously silky formulation, among other end products, which exhibit good fragrance solubility, clarity and product stability. The long-lasting qualities of essence fragrances of top notes in particular, are a benefit of the disclosed fragrance carrier formulation.

The anhydrous alcohol-free fragrance carrier presented herein may be employed by a user to obtain the benefits of the fragrance product formulation without the disadvantages associated with using alcohol in such fragrance-based products, which can be irritating to the skin, and also evaporates the fragrance rather quickly, in particular the top notes. Such evaporation of the top notes of the fragrance, in particular, can rapidly change the overall original balance of the total fragrance.

In certain embodiments, the disclosed subject matter is suited for use as a carrier for oil-infused perfume compositions, such as perfume or cologne oil end products, for example, rollerball fragrance products, dropper-based end products, liquid-pump dispensers, and even formulations as creams, gels, lotions, and the like, especially for users with sensitive skin. Such products can be used on one's skin and can achieve silky moisturizing, with silkening nourishment capable of a highly concentrated expression of exquisite or luxurious quality fragrance. Such end-product is also fully absorbed prior to any delicate fabrics coming into contact with one's skin.

For purpose of explanation and illustration, and not limitation, exemplary embodiments of the anhydrous silky fragrance carrier composition in accordance with the disclosed subject matter are described herein.

The disclosed subject matter relates to an anhydrous silky fragrance carrier formulation containing perfume or cologne ingredients used in perfumery. These ingredients belong to chemical classes such as the alcohols, aldehydes, ketones, esters, ethers, terpenes. These ingredients must be essentially polar to be soluble in the fragrance carrier.

The term "fragrance product" is used throughout this application to refer to any compound, composition, product, and the like that is intended to be applied to a person's body to improve the person's skin appearance, fragrance, attractiveness, silky luxurious feel, and the like. This includes, without limitation, perfumed-based makeup, creams, and/or gels, and/or fragrances such as perfume, cologne, fragrant perfume oil products (rollerball or dropper), aftershaves, cosmetic creams and/or other cosmetic formulations, and the like.

In accordance with the disclosed subject matter, as embodied and broadly described herein, the anhydrous alcohol-free silky fragrance carrier formulation is composed of preferably, in example embodiments of the following:
- about 1 to 40 wt. % of perfume compound(s), more preferably 10 to 30 wt. %;
- about 10 to 50 wt. % of triethyl citrate, more preferably 10 to 35 wt. %;
- about 10 to 40 wt. % of ethyl hexyl glycerin, more preferably 10 to 25 wt. %;
- Optionally, some diluent(s) such as propylene glycol, 1,3 propanediol, dipropylene glycol, isopropyl myristate, isopropyl palmitate and/or combination may be added; and
- One or more Natural oil(s) with skin soothing, moisturizing action may also optionally be added of up to 5 wt. %.

While in certain disclosed embodiments, some diluent(s) such as propylene glycol, 1, 3 propanediol, dipropylene glycol, isopropyl myristate, isopropyl palmitate or combinations, may be added to the above-described base formulations (i.e., 1 to 40 wt. % of perfume compound(s), preferably 10 to 30 wt. %; 10 to 50 wt. % of triethyl citrate, preferably 10 to 35 wt. %; and 5 to 40 wt. % ethyl hexyl glycerin, preferably 5 to 25 wt. %). An example embodiment comprises the combination of the same above-listed main ingredients.

In accordance with a preferred embodiment of the disclosed subject matter, the anhydrous alcohol-free silky fragrance carrier formulation is more preferably composed of:
- 1 to 40 wt. % of perfume compound(s), preferably 10 to 30 wt. %;
- 10 to 50 wt. % of triethyl citrate, preferably 10 to 35 wt. %;
- 5 to 40 wt. % of ethyl hexyl glycerin, preferably 5 to 25 wt. %;
- Optionally some diluent(s) such as propylene glycol, 1,3 propanediol, dipropylene glycol, isopropyl myristate, isopropyl palmitate or a combination may be added; and
- One or more Natural oil(s) with skin soothing, moisturizing action may also optionally be added of up to 5 wt. %.

The amount and/or type of fragrance notes and/or essences contained in the perfume compounds according to the present disclosure may be selected by a person skilled in the art according to the desired fragrance intensity. The variety of fragrance notes is described in further detail hereinbelow.

In accordance with an embodiment of the disclosed subject matter, and more particularly, triethyl citrate which is an ester of citric acid from citrus fruits is one of the main ingredients for the disclosed fragrance carrier formulation. Triethyl citrate is a colorless, nonflammable clear liquid. It is quite widely used by the major perfumery manufacturers as a diluent for ingredients, including polar ingredients. Triethyl citrate (other names are ethyl citrate, E1505, Citric acid ethyl ester) is represented by the formula (1) below:

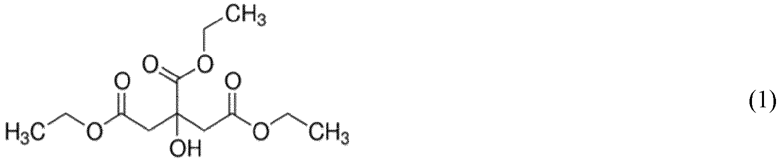

Hence, triethyl citrate, can be used as a diluent for the ingredients in the silky fragrance carrier formulation. Triethyl citrate is a colorless, odorless liquid compound used as an additive. The chemical molecular formula is C₁₂H₂₀O₇ or (CH₂COOC₂H₅)₂COHCOOC₂H₅ (with preferred International Union of Pure and Applied chemistry (IUPACP name is Triethyl 2-hydroxypropane-1,2,3-tricarboxylate). A linear formula for Triethyl citrate is further represented as HOC(COOC₂H₅)(CH₂COOC₂H₅)₂.

Triethyl Citrate is considered a versatile cosmetic ingredient with excellent skincare properties manufactured from nature-derived agro resources. It is a non-toxic, eco-friendly and bio-degradable ingredient made from 100 % renewable carbon source. It is not shown to be an irritant to the skin of humans and laboratory animals. In dilute form, it shows no sensitizing potential in humans. Acute toxicity was exhibited as low by the oral, dermal and inhalation routes in laboratory animals. Triethyl citrate is produced by a process of esterification of citric acid with ethanol. It is naturally present in small amounts in wine and is easily metabolized by the human body. In cosmetics, this ingredient is frequently used in deodorants formulated as alternatives to aluminum salts. Because of its ability to inhibit enzymatic decomposition of sweat components, it is considered an excellent active ingredient in the fight against body odor. Triethyl citrate also has antibacterial properties. It is authorized in organic as well. Hence, triethyl citrate is considered a desirable ingredient for perfumes and aromatic raw materials.

Another ingredient included in embodiments of the formulation of the disclosed anhydrous silky fragrance carrier, is ethyl hexyl glycerin, which is a glyceryl ether. Ethyl hexyl glycerin is a colorless, clear liquid preservative derived from vegetable glycerin, often coconut or a vegetable-derived oil. In addition, ethyl hexyl glycerin in example embodiments of the disclosed fragrance carrier formulation, acts as a booster and fixative for fragrances.

The ingredient, ethyl hexyl glycerin is represented by formula (2) below:

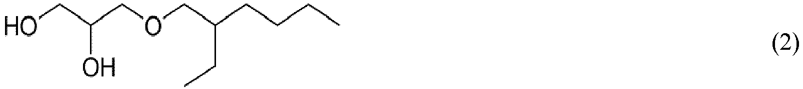

More particularly, ethyl hexyl glycerin is a chemical compound that is a direct derivative of glycerine. It is derived from synthetic raw materials. It is generally deemed to be considered very safe and a non-irritant to skin. Further, ethyl hexyl glycerin is known for its deodorizing properties, acts as a preservative, and also as an active skin conditioning agent. It has extensive use as an alternative to parabens in cosmetic preparations since parabens have been time and again, identified as a source of several critical diseases in humans. Ethyl hexyl glycerin also is considered an active surfactant. When it comes to using the chemical as a preservative, it works really well, even in small amounts and makes it an economical choice for mass producing cosmetic plants. In many types of research, it has proven for use as an enhancer for cosmetic preparations, thereby replacing parabens. The chemical also arrests the growth of bacteria that causes body odor without disturbing the beneficial bacteria present on the human skin in any skin or scent-based formulations. Ethyl hexyl glycerin is known for its ability to boost the efficiency of all known traditional preservatives. Further, ethyl hexyl glycerin also acts as a stabilizer in cosmetic or fragrance preparations that keeps microbial life in check. The chemical is highly pure, colorless and carries a mild odor that is negligible, except for perhaps, a well-trained nose.

In a moisturizing formulation, ethyl hexyl glycerin is an impressive skin moisturizing agent. It is known to improve the overall texture of cosmetic formulations for the skin. In example embodiments comprising fragrance-based formulations, ethyl hexyl glycerin helps in boosting the effects of fragrance ingredients and properly stabilizes and/or fixes the fragrance ingredients to the formulations. In certain implementations, ethyl hexyl glycerin also helps to reduce the surface tension between the various liquids and oils in beauty products. The chemical is a commonly used ingredient in skincare products. It is also known to improve the skin feel of cosmetic and/or fragrance-based formulations for skin. It essentially serves as a multi-functional ingredient in the cosmetic industry. Ethyl hexyl glycerin also boosts antimicrobial efficacy and enables lower concentrations of typically used preservatives including parabens or triclosan, and thus, is considered a multifunctional preservative component with emollient and humectants functionality.

The chemical formula for ethyl hexyl glycerin is C₁₁H₂₄O₃. The IUPAC name is 3-[(2-Ethylhexyl)oxy]-1,2-propanediol. Another name for same chemical is Octoxyglycerin.

In certain embodiments of the disclosed fragrance carrier formulation, other fragrance diluents such as glycols (dipropylene glycol, 1,3 propanediol, propylene glycol), esters (isopropyl myristate, isopropyl palmitate), natural oils (vegetable oil, jojoba oil,), glucose ethers (propoxylated methyl glucose ether), and/or glycerin esters (caprylic/capryl glycerin) may be added to the formulation.

In the fragrance industry generally, flavor or fragrance formulations represent a mixture of aroma compound or in some cases a single compound is used to create a specific smell for use in a variety of consumer goods products. Formulations are generally created by specially trained scientists, flavorists or perfumers, whose job combines scientific knowledge of the chemical palette with creativity to develop new and distinctive fragrant creations. The perfumer uses their knowledge of the available chemical ingredients to create a formula and compound which is then submitted to the client for testing. Several iterations, with even feedback from the client(s), may be conducted before the right or desired formulation is found. Sensory tests may be further conducted to determine consumer acceptance of the formulation and further investigation into "sensory space".

As used herein, "fragrance" is used to indicate any odoriferous material that is generally considered pleasant to fragrance consumers. Any fragrance that is cosmetically acceptable may be used in the composition. For example, the fragrance may be one that is a liquid at room temperature. In preferred embodiment the perfume compounds including fragrance notes, are present from about 1 to 40 wt. %, and more preferably between 10 to 30 wt. %.

A wide variety of chemicals are known as fragrances, including aldehydes, ketones, and esters. More commonly, naturally occurring plant and animal oils and exudates comprising complex mixtures of various chemical components are known for use as fragrances. Non-limiting examples of the fragrances useful herein include pro-fragrances such as acetal pro-fragrances, ketal pro-fragrances, ester pro-fragrances, hydrolyzable inorganic-organic pro-fragrances, and mixtures thereof. The fragrances may be released from the pro-fragrances in a number of ways. For example, the fragrance may be released as a result of simple hydrolysis, by a shift in an equilibrium reaction, by a pH-change, or by enzymatic release. Embodiments of the present disclosure helps prevent and/or slow down any such deterioration to original fragrance notes. The fragrances may be relatively simple in their chemical make-up, comprising a single chemical, or may comprise highly sophisticated complex mixtures of natural and synthetic chemical components, all chosen to provide any desired odor.

Generally speaking, example fragrances may have a boiling point (BP) of about 500° C. or lower, about 400° C. or lower, or about 350° C. or lower. The BP of many fragrances are disclosed in Perfume and Flavor Chemicals (Aroma Chemicals), Steffen Arctander (1969). The C log P value of the fragrances may be about 0.1 or greater, about 0.5 or greater, about 1.0 or greater, or about 1.2 or greater. As used herein, "C log P" means the logarithm to the base 10 of the octanol/water partition coefficient. The C log P can be readily calculated from a program called "C LOG P" which is available from Daylight Chemical Information Systems Inc., Irvine Calif., USA. Octanol/water partition coefficients are described in more detail in U.S. Pat. No. 5,578,563.

Suitable fragrances are also disclosed in U.S. Pat. Nos. 4,145,184, 4,209,417, 4,515,705, and 4,152,272. Non-limiting examples of fragrances include animal fragrances such as musk oil, civet, castoreum, ambergris, plant fragrances such as nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

Other examples of suitable fragrances include, but are not limited to, chemical substances such as acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, α-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-α-naphthyl ketone, methylphenylcarbinyl acetate, mugetanol, γ-nonalactone, octanal, phenyl ethyl acetate, phenylacetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, γ-undecalactone, undecenal, vanillin, veloutone, verdox, and mixtures thereof.

Examples of fragrance products of natural origin include, but are not limited to, essential oils extracted from different parts of plants (flowers, stems, leaves, fruits, bark, roots, woody parts, herbs, needles, sap and gums), resinoids, concretes, or absolutes obtained from them. Preferred examples of naturally occurring complex products include white grass mugwort oil (Artemisia Herba-Alba), Pogostemon Cablin leaf oil (Pogostemon Cablin Leaf Oil), Citrus nobilis bark oil (Citrus nobilis peel oil), Barosma Betulina Leaf Oil (Barosma Betulina Leaf Extract), Citrus Limon Peel extract, Eucalyptus globulus leaf oil, Dipterocarpus turbinatus oil ( Dipterocarpus turbinatus Balsam Oil), Pogostemon cablin leaf oil, Rosmarinus officinalis leaf oil, Juniperus virginiana oil, Wild mint leaf oil (Mentha Arvensis), Spearmint leaf oil (Mentha viridis) Citrus Aurantium Dulcis Bark, Citrus Aurantium Dulcis Bark Extract, Mediterranean Cypress Leaf Oil (Cupressus sempervirens), Patchouli Leaf Oil (Pogostemon cablin), Cedar T exas (Juniperus Mexicana) and Lavandula Hybrid Oil (Lavandula Hybrida).

The one or more of or all odor essences contained in an alcohol-free fragrance carrier composition can also be contained in a perfume oil microemulsion according to the present disclosure in the form of extracts from natural raw materials, for example in the form of essential oils, concretes, absolutes, resins, resinoids, balsams or tinctures, preferably those from the group consisting of: ambergris tincture; amyris oil; angelica seed oil; angelica root oil; anise oil; valerian oil; basil oil; tree moss absolute; bay oil; mugwort oil; benzoic resin; bergamot oil; beeswax absolute; birch tar oil; bitter almond oil; savory oil; bucho leaf oil; cabreuva oil; cade oil; calamus oil; camphor oil; cananga oil; cardamom oil; cascarilla oil; cassia oil; cassia absolute; castoreum absolute; cedar leaf oil; cedar wood oil; cistus oil; citronellol; lemon oil; copaiba balsam; copaiba balsam oil; coriander oil; costus root oil; cumin oil; cypress oil; davana oil; dill oil; dill seed oil; eau de brouts absolute; oak moss absolute; elemi oil; tarragon oil; eucalyptus-citriodora oil; eucalyptus oil; fennel oil; spruce needle oil; galbanum oil; galbanum resin; geranium oil; grapefruit oil; guaiac wood oil; gurjun balsam; gurjun balsam oil; helichrysum absolute; helichrysum oil; ginger oil; iris root absolute; iris root oil; jasmine absolute; calamus oil; blue chamomile oil; Roman chamomile oil; carrot seed oil; cascarilla oil; pine needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil pressed; linalool oil; litsea-cubeba oil; bay leaf oil; mace oil; marjoram oil; mandarin oil; massoy bark oil; mimosa absolute; musk seed oil; musk tincture; muscatel-sage oil; nutmeg oil; myrrh absolute; myrrh oil; myrtle oil; clove leaf oil; clove blossom oil; neroli oil; olibanum absolute; olibanum oil; opopanax oil; orange blossom absolute; orange oil; origanum oil; palmarosa oil; patchouli oil; perilla oil; Peru balsam oil; parsley leaf oil; parsley seed oil; petitgrain oil; peppermint oil; pepper oil; pimento oil; pine oil; poley oil; rose absolute; rosewood oil; rose oil; rosemary oil; Dalmatian sage oil; Spanish sage oil; sandalwood oil; celery seed oil; spike lavender oil; star anise oil; styrax oil; tagetes oil; fir-needle oil; tea tree oil; turpentine oil; thyme oil; tolu balsam; tonka absolute; tuberose absolute; vanilla extract; violet leaf absolute; verbena oil; vetiver oil; Juniper berry oil; wine lees oil; wormwood oil; wintergreen oil; ylang-ylang oil; hyssop oil; civet absolute; cinnamon leaf oil; cinnamon bark oil and fractions thereof, or ingredients isolated therefrom.

The fragrance formulation according to the present disclosure may comprise ingredients of natural or in certain embodiments, of synthetic origin. The choice of this perfume depends on the one hand on the desired odor effect and on the other hand on the nature of the product that contains it.

The present invention solves the different problems and drawbacks mentioned previously, by means of the disclosed anhydrous non-alcoholic (or alcohol-free) silky fragrance formulation with an exhibited good clarity, solubility, aesthetically pleasing quality, with a luxuriously smooth feel, including a non-tacky or non-sticky rub-out effect when applied to the skin. The carrier for the disclosed formulation is composed of a combination of triethyl citrate and ethyl hexyl glycerin.

In embodiments of the anhydrous silky fragrance carrier formulation embodied in a cosmetic end-product, the composition includes one or more lipophilic actives such as anti-aging, anti-wrinkle, vitamins, sunscreen, anti-acne ingredients, and fragrance.

In embodiments, one or more emollients can be used to moisturize the skin or to increase the miscibility of the actives and fragrance in other embodiments. Moreover, in accordance with certain embodiments, a natural preservative enhancer (for example, zemea) and/or some pigments can be added to the formula to naturally preserve the product from bacteria contamination and to improve the aesthetics. The anhydrous silky fragrance carrier formulation, in accordance with the disclosed subject matter, can be used with a variety of cosmetic or fragrance products such as creams, gels, and lotions without causing reactions with polymers.

The disclosed subject matter allows the anhydrous silky cosmetic and/or fragrance product to contain both no alcohol and water. Therefore, in some embodiments, the addition of about 1 to 90 percent by weight non-silicone fluid can be used to adjust the viscosity and, when a volatile fluid is used, to get a dry feel. Furthermore, in some embodiments, one or more emollients can be added to the anhydrous silky fragrance carrier formulation and/or cosmetic product to increase the miscibility of the active or fragrance oil, to keep the skin moist and flexible, or to help to prevent cracks.

In certain embodiments, of the fragrance product, dipropylene glycol, hedione, isopropyl myristate, isopropyl palmitate, triethyl citrate or benzyl benzoate are generally the diluents used to make fragrance. However, dipropylene glycol and triethyl citrate are diluents considered more polar than the others.

In embodiments, an oil such as a naturally derived vegetable-based oil, which is a natural or cosmetic "active" at 0.01 - 1.0 %. can be added to the anhydrous silky cosmetic fragrance carrier formulation to moisturize the skin or to increase the miscibility of the active or fragrance product. In certain embodiments, for example, when a high level of non-silicone volatile fluid is used in the formulation. Examples of suitable emollients are isononyl isononanoate, Aloe Vera, PPG-20 methyl glucose ether distearate, caprylic/capric triglyceride, neopentyl glycol diheptanoate, jojoba oil, isopropyl isostearate, and polyisobutene.

Additionally, in some embodiments, one or more preservatives, pigments, or combinations thereof, can be added to the anhydrous silky fragrance carrier formulation to preserve the product from bacteria contamination and to improve the aesthetics. The preservative-free quality to the disclosed fragrance formulation since many of the preservatives currently used in cosmetics are being banned by many cosmetic customers. So, for example, materials such as parabens, formaldehyde, and formaldehyde donors and many others, appear on industry watchdog lists.

Being preservative-free is another desirable quality of the present fragrance carrier formulation. Including high levels of water or creating water-based formulations often will help preserve the fragrance top notes, but may likely cause the formulation to have instability or sticky feel in character. The presently disclosed formulation is not only alcohol, silicone, and water free, but exhibits excellent fragrance character, aesthetics and skin feel with an additional luxuriously moisturizing quality thereto. The result is a preservative-free formulation, VOC free, very silky and non-oily feel, hence, lending itself to an overall luxurious and/or silky feel with an overall stable fragrance character, in particular preservation of the fragrance top notes, which can often evaporate or deteriorate otherwise. The present disclosure can hold high levels of fragrance for up to 30%. In certain embodiments, the % value of holding high levels of fragrance can be higher than 30%, without affecting the fragrance character or aesthetics. The disclosed carrier formulation achieves excellent fragrance duration of up to 8 hours, and in certain embodiments, even longer.

The present fragrance carrier formulation is preservative-free, preserved naturally not only because of its anhydrous properties, but also because of naturally derived zemea (1,3 propanediol) (or similar ingredient) which has natural bacteriostatic properties. In particular, zemea delivers a multifunctional, preservative-boosting humectant and such ingredient delivers high performance in a variety of applications - including cosmetics and fragrances. Zemea is considered sustainably sourced and generates up to 40% less greenhouse gas emissions over its life.

The viscosity range of the anhydrous alcohol-free silky fragrance carrier formulation is about 20 to 55 mPa·S (20-55 centipoise, cP) in certain disclosed embodiments. This light feeling preparation exhibits a texture and feel of a slightly viscous "oil" without being greasy. It has a luxuriously silky feel when applied to the skin with lasting fragrance notes that is considered attractive to consumers and which in certain disclosed embodiments, can comprise a mixture of one or more fragrant essential oils, aroma compounds, fixatives and/or solvents.

Exemplary embodiments and/or examples of the anhydrous silky fragrance carrier formulation in accordance with the present disclosure will now be described hereinbelow.

### EXAMPLES:

The following ingredients were blended at room temperature in the indicated amount.

### EXAMPLE 1

**Table 1** lists the ingredients and composition of three different example embodiments of the anhydrous silky alcohol-free fragrance carrier formulation in accordance with the present disclosure. The different formulations shown for each sample were clear with a good olfactive rendition without alteration of the fragrance notes, and hence, retaining the original fragrance character.

**TABLE 1:**

| Sample # | Fragrance wt. % | Triethyl Citrate wt.% | Dipropylene Glycol wt. % | Ethylhexyl Glycerin wt. % | 1,3 propanediol wt. % | Natural oil wt. % |
|---|---|---|---|---|---|---|
| 1 | 30 | 30 | 20 | 15 | 5 | - |
| 2 | 30 | 25 | 25 | 14.9 | 5 | 0.1 |
| 3 | 30 | 20 | 25 | 19.9 | 5 | 0.1 |

### EXAMPLE 2:

**Table 2** lists the ingredients and composition of an example of the anhydrous silky alcohol-free fragrance formulation, in accordance with an embodiment of the present disclosure.

**TABLE 2:**

| **Phase** | **Description** | **Percent wt.%** | **Qty Req.** |
|---|---|---|---|
| Oil | Ebony santal mod 4 oil | 30 | 30 |
| Stabilizer | Dipropylene Glycol | 25 | 25 |
| Base 1 | Ethylhexyl Glycerin | 19.9 | 19.9 |
| Base 2 | Base/Triethyl Citrate | 20 | 20 |
| Stabilizer | Zemea Propanediol | 5 | 5 |
| Oil | Natural Oil | 0.1 | 0.1 |

**Table 2** indicates the ingredients and composition of fragrance diluents, in accordance with an embodiment of the present disclosure. Each of the ingredients including the shown additive Natural oil, were blended and result a fragrance carrier formulation that brought consumers a heightened fragrance experience while nourishing their skin as well.

Example embodiments include a rose perfume oil formulation comprising the fragrance notes of fruity, floral, and/or hints of spice warmed by woods. Another embodiment comprises a citrus perfume oil formulation. Such example formulation includes notes of effervescent orange zest, tart pink grapefruit, and/or juicy mandarin with floral neroli and cedarwood. Yet another embodiment, is a white floral perfume oil formulation which includes fragrant notes of jasmine with an intense Jasmine absolute surrounded by red fruits, sparkling citrus, and/or spice. In yet another embodiment, a vanilla perfume oil formulation includes fragrance notes with the warm essence of vanilla bean, the subtle floralcy of vanilla orchid, and/or a hint of coconut. In yet another example embodiment, is an Australian sandalwood perfume oil formulation with concentrated fragrance essence of sandalwood, fresh florals, and/or vetiver. In yet another example embodiment, is rose, lush fruits, and/or woods. Other contemplated fragrance notes variations (including top, middle and/or base fragrance notes) are intended by the disclosed subject matter and should be interpreted as limited to the above example formulations. The most volatile smells are described as "top" notes to the least volatile ones as the "base" notes (often also called the "dry-down"). Usually, the main theme of the fragrance will be described as the middle note, or what is also called the "heart" of the fragrance. The contemplated embodiments, comprise a fragrance formulation capable of providing the requisite stability and lasting quality to the top, middle and/or base notes of the fragrance carrier.

## Claims

1. An anhydrous alcohol-free fragrance carrier composition comprising:
at least one perfume compound, present in an amount of about 1 to 40 percent by weight relative to a total weight of the composition;
triethyl citrate, present in an amount of about 10 to 50 percent by weight relative to the total weight of the composition; and
ethyl hexyl glycerin in an amount of about 10 to 40 percent by weight relative to the total weight of the composition.

2. The anhydrous alcohol-free fragrance carrier composition of claim 1, further comprising one or more diluents, optionally wherein the one of more diluents is selected from the group consisting of: propylene glycol, 1, 3 propanediol, dipropylene glycol, ispopropyl myristate, isopropyl palmitate and combinations thereof.

3. The anhydrous alcohol-free fragrance carrier composition of claim 1, further comprising one or more fragrance diluent(s).

4. The anhydrous alcohol-free fragrance carrier composition of claim 3, wherein the one or more fragrance diluent(s) is selected from the group consisting of: glycol(s), natural oil(s), glucose ether(s), glycerin ester(s), and combinations thereof.

5. The anhydrous alcohol-free fragrance carrier composition of claim 4, wherein the one or more glycol(s) is selected from the group consisting of: dipropylene glycol, 1,3 propanediol, propylene glycol, and combinations thereof, or wherein the one or more natural oils is selected from the group consisting of: natural oil, jojoba oil, a vegetable-derived oil, and combinations thereof, or wherein the glucose ether(s) comprises a propoxylated methyl glucose ether, or wherein the glycerin esther comprises caprylic or

6. The anhydrous alcohol-free fragrance carrier composition of claim 1, further comprising an anti-aging ingredient, an anti-wrinkle ingredient, a vitamin, a sunscreen, an anti-acne ingredient, or a fragrance oil, or further comprising a preservative-boosting humectant and/or stabilizer.

7. The anhydrous alcohol-free fragrance carrier composition of claim 1, further comprising at least one emollient, optionally wherein the emollient comprises isononyl isononanoate, Aloe Vera, PPG-20 methyl glucose ether distearate, caprylic/capric triglyceride, neopentyl glycol diheptanoate, jojoba oil, isopropyl isostearate, or polyisobutene.

8. The anhydrous alcohol-free fragrance carrier composition of claim 1, wherein the composition is VOC-free, or wherein the composition is preservative-free.

9. The anhydrous alcohol-free fragrance carrier composition of claim 1, wherein the at least one perfume compound, is present in an amount of about 10 to 30 percent by weight relative to the total weight of the composition, or wherein the triethyl citrate, is present in an amount of about 10 to 35 percent by weight relative to the total weight of the composition, or wherein the ethyl hexyl glycerin is present in an amount of about 10 to 25 percent by weight relative to the total weight of the composition, or wherein the ethyl hexyl glycerin is present in an amount of about 5 to 25 percent by weight relative to the total weight of the composition.

10. The anhydrous alcohol-free fragrance carrier composition of claim 1, wherein the composition has a viscosity between 20 to 55 mPa·S (20-55 centipoise, cP).

11. The anhydrous alcohol-free fragrance carrier composition of claim 1, wherein the fragrance notes comprise one or more of: jasmine, red berries, bergamot, spice, vanilla bean, vanilla orchid, coconut, sandalwood, floral leaves, vetiver, rose, dark fruits, woods, orange zest, tart pink grapefruit, juicy mandarin, floral neroli and/or cedarwood, or combinations thereof.

12. The anhydrous alcohol-free fragrance carrier composition of claim 1, wherein the composition is silicone-free.

13. The anhydrous alcohol-free fragrance carrier composition of claim 1, wherein the composition further comprises one or more natural oils with skin soothing, moisturizing action of up to 5% present by weight relative to the total weight of the composition.

14. An anhydrous alcohol-free fragrance carrier composition comprising:
at least one perfume compound, present in an amount of about 10 to 30 percent by weight relative to a total weight of the composition;
triethyl citrate, present in an amount of about 10 to 35 percent by weight relative to the total weight of the composition; and
ethyl hexyl glycerin in an amount of about 5 to 25 percent by weight relative to the total weight of the composition.

## Patentansprüche

1. Eine wasserfreie, alkoholfreie Duftstoffträger-Zusammensetzung, umfassend:
mindestens ein Parfümbestandteil, der in einer Menge von etwa 1 bis 40 Gewichtsprozent, bezogen auf das Gesamtgewicht der Zusammensetzung, vorhanden ist;
Triethylcitrat, das in einer Menge von etwa 10 bis 50 Gewichtsprozent, bezogen auf das Gesamtgewicht der Zusammensetzung, vorhanden ist; und
Ethylhexylglycerin, das in einer Menge von etwa 10 bis 40 Gewichtsprozent, bezogen auf das Gesamtgewicht der Zusammensetzung, vorhanden ist.

2. Die wasserfreie, alkoholfreie Duftstoffträger-Zusammensetzung nach Anspruch 1, die ferner ein oder mehrere Verdünnungsmittel umfasst, optional wobei das eine oder die mehreren Verdünnungsmittel ausgewählt sind aus der Gruppe bestehend aus: Propylenglykol, 1,3-Propandiol, Dipropylenglykol, Isopropylmyristat, Isopropylpalmitat und Kombinationen davon.

3. Die wasserfreie, alkoholfreie Duftstoffträger-Zusammensetzung nach Anspruch 1, ferner umfassend ein oder mehrere Duftstoffverdünnungsmittel.

4. Die wasserfreie, alkoholfreie Duftstoffträger-Zusammensetzung nach Anspruch 3, wobei das eine oder die mehreren Duftstoffverdünnungsmittel ausgewählt sind aus der Gruppe bestehend aus: Glykol(e), natürliche Öle, Glukoseether, Glycerinester und Kombinationen davon.

5. Die wasserfreie, alkoholfreie Duftstoffträger-Zusammensetzung nach Anspruch 4, wobei das eine oder die mehreren Glykole ausgewählt sind aus der Gruppe bestehend aus: Dipropylenglykol, 1,3-Propandiol, Propylenglykol und Kombinationen davon, , oder wobei das eine oder die mehreren natürlichen Öle ausgewählt sind aus der Gruppe bestehend aus: natürlichem Öl, Jojobaöl, einem pflanzlichen Öl, und Kombinationen davon, , oder wobei der Glukoseether einen propoxylierten Methylglucoseether umfasst, oder wobei der Glycerinester Caprylsäure- oder Caprylglycerin umfasst.

6. Die wasserfreie, alkoholfreie Duftstoffträger-Zusammensetzung nach Anspruch 1, die ferner einen anti-Aging Wirkstoff, einen anti-Falten Wirkstoff, ein Vitamin, ein Sonnenschutzmittel, einen anti-Akne Wirkstoff oder ein Duftöl, oder ferner einen konservierungsverstärkenden Feuchthaltestoff und/oder Stabilisator umfasst.

7. Die wasserfreie, alkoholfreie Duftstoffträger-Zusammensetzung nach Anspruch 1, die ferner mindestens einen Weichmacher umfasst, optional wobei der Weichmacher Isononylisononanoat, Aloe Vera, PPG-20-Methylglucoseetherdistearat, Capryl-/Capric-Triglycerid, Neopentylglykoldiheptanoat, Jojobaöl, Isopropylisostearat oder Polyisobuten umfasst.

8. Die wasserfreie, alkoholfreie Duftstoffträger-Zusammensetzung nach Anspruch 1, wobei die Zusammensetzung VOC-frei ist, oder wobei die Zusammensetzung frei von Konservierungsstoffen ist.

9. Die wasserfreie, alkoholfreie Duftstoffträger-Zusammensetzung nach Anspruch 1, wobei der mindestens eine Parfümbestandteil in einer Menge von etwa 1 bis 30 Gewichtsprozent, bezogen auf Gesamtgewicht der Zusammensetzung, vorhanden ist, oder wobei das Triethylcitrat in einer Menge von etwa 10 bis 35 Gewichtsprozent, bezogen auf das Gesamtgewicht der Zusammensetzung vorhanden ist, oder wobei das Ethylhexylglycerin in einer Menge von etwa 10 bis 25 Gewichtsprozent, bezogen auf das Gesamtgewicht der Zusammensetzung, vorhanden ist, oder wobei das Ethylhexylglycerin in einer Menge von etwa 5 bis 25 Gewichtsprozent, bezogen auf das Gesamtgewicht der Zusammensetzung, vorhanden ist.

10. Die wasserfreie, alkoholfreie Duftstoffträger-Zusammensetzung nach Anspruch 1, wobei die Zusammensetzung eine Viskosität zwischen 20 und 55 mPa·S (20-55 centipoise, cP) aufweist.

11. Die wasserfreie, alkoholfreie Duftstoffträger-Zusammensetzung nach Anspruch 1, wobei die Duftstoffnoten eins oder mehr umfassen von: Jasmin, rote Beeren, Bergamotte, Gewürze, Vanilleschote, Vanilleorchidee, Kokosnuss, Sandelholz, Blütenblätter, Vetiver, Rose, dunkle Früchte, Hölzer, Orangenschale, säuerliche rosa Grapefruit, saftige Mandarine, blumiges Neroli- und/oder Zedernholz oder Kombinationen davon.

12. Die wasserfreie, alkoholfreie Duftstoffträger-Zusammensetzung nach Anspruch 1, wobei die Zusammensetzung silikonfrei ist.

13. Die wasserfreie, alkoholfreie Duftstoffträger-Zusammensetzung nach Anspruch 1, wobei die Zusammensetzung ferner ein oder mehrere natürliche Öle mit hautberuhigender, feuchtigkeitsspendender Wirkung von bis zu 5 Gewichtsprozent, bezogen auf das Gesamtgewicht der Zusammensetzung, umfasst.

14. Eine wasserfreie, alkoholfreie Duftstoffträger-Zusammensetzung , umfassend:
mindestens ein Parfümbestandteil, der in einer Menge von etwa 10 bis 30 Gewichtsprozent, bezogen auf Gesamtgewicht der Zusammensetzung, vorhanden ist;
Triethylcitrat, das in einer Menge von etwa 10 bis 35 Gewichtsprozent, bezogen auf das Gesamtgewicht der Zusammensetzung, vorhanden ist; und
Ethylhexylglycerin, das in einer Menge von etwa 5 bis 25 Gewichtsprozent, bezogen auf das Gesamtgewicht der Zusammensetzung, vorhanden ist.

## Revendications

1. Une composition anhydre porteuse de fragrance sans alcool comprenant :
au moins un composé parfumé, présent en une quantité d'environ 1 à 40 pour cent en poids par rapport à un poids total de la composition ;
du citrate de triéthyle, présent en une quantité d'environ 10 à 50 pour cent en poids par rapport au poids total de la composition ; et
de l'éthylhexylglycérine en une quantité d'environ 10 à 40 pour cent en poids par rapport au poids total de la composition.

2. La composition anhydre porteuse de fragrance sans alcool selon la revendication 1, comprenant en outre un ou plusieurs diluants, facultativement dans laquelle les un ou plusieurs diluants sont choisis dans le groupe consistant en : propylène-glycol, 1,3-propanediol, dipropylène-glycol, myristate d'isopropyle, palmitate d'isopropyle, et les combinaisons de ceux-ci.

3. La composition anhydre porteuse de fragrance sans alcool selon la revendication 1, comprenant en outre un ou plusieurs diluants de fragrance.

4. La composition anhydre porteuse de fragrance sans alcool selon la revendication 3, dans laquelle les un ou plusieurs diluants de fragrance sont choisis dans le groupe consistant en : glycol(s), huile(s) naturelle(s), éther(s) de glucose, ester(s) de glycérine, et les combinaisons de ceux-ci.

5. La composition anhydre porteuse de fragrance sans alcool selon la revendication 4, dans laquelle les un ou plusieurs glycols sont choisis dans le groupe consistant en : dipropylène-glycol, 1,3-propanediol, propylène-glycol, et les combinaisons de ceux-ci, ou dans laquelle les une ou plusieurs huiles naturelles sont choisies dans le groupe consistant en : huile naturelle, huile de jojoba, huile d'origine végétale, et les combinaisons de celles-ci, ou dans laquelle les éthers de glucose comprennent un méthylglucose éther propoxylé, ou dans laquelle l'ester de glycérine comprend de la glycérine caprylique ou de capryle.

6. La composition anhydre porteuse de fragrance sans alcool selon la revendication 1, comprenant en outre un ingrédient anti-vieillissement, un ingrédient antirides, une vitamine, un écran solaire, un ingrédient anti-acné, ou une huile fragrance, ou comprenant en outre un humectant et/ou stabilisant renforçant les conservateurs.

7. La composition anhydre porteuse de fragrance sans alcool selon la revendication 1, comprenant en outre au moins un émollient, facultativement dans laquelle l'émollient comprend de l'isononanoate d'isononyle, de l'aloe vera, du distéarate de méthylglucose éther PPG-20, du triglycéride caprylique/caprique, du diheptanoate de néopentylglycol, de l'huile de jojoba, de l'isostéarate d'isopropyle ou polyisobutène.

8. La composition anhydre porteuse de fragrance sans alcool selon la revendication 1, dans laquelle la composition est exempte de COV, ou dans laquelle la composition est exempte de conservateur.

9. La composition anhydre porteuse de fragrance sans alcool selon la revendication 1, dans laquelle l'au moins un composé parfumé est présent en une quantité d'environ 10 à 30 pour cent en poids par rapport au poids total de la composition, ou dans laquelle le citrate de triéthyle est présent en une quantité d'environ 10 à 35 pour cent en poids par rapport au poids total de la composition, ou dans laquelle l'éthylhexylglycérine est présente en une quantité d'environ 10 à 25 pour cent en poids par rapport au poids total de la composition, ou dans laquelle l'éthylhexylglycérine est présente en une quantité d'environ 5 à 25 pour cent en poids par rapport au poids total de la composition.

10. La composition anhydre porteuse de fragrance sans alcool selon la revendication 1, dans laquelle la composition présente une viscosité entre 20 et 55 mPa·S (20 à 55 centipoise, cP).

11. La composition anhydre porteuse de fragrance sans alcool selon la revendication 1, dans laquelle les notes de fragrance comprennent un ou plusieurs parmi : jasmin, baies rouges, bergamote, épice, fève de vanille, orchidée vanille, noix de coco, bois de santal, feuilles florales, vétiver, rose, fruits foncés, bois, zeste d'orange, pamplemousse rose acidulé, mandarine juteuse, néroli floral et/ou bois de cèdre, ou les combinaisons de ceux-ci.

12. La composition anhydre porteuse de fragrance sans alcool selon la revendication 1, dans laquelle la composition est exempte de silicone.

13. La composition anhydre porteuse de fragrance sans alcool selon la revendication 1, dans laquelle la composition comprend en outre une ou plusieurs huiles naturelles ayant une action apaisante et hydratante pour la peau, présente jusqu'à 5 % en poids par rapport au poids total de la composition.

14. Une composition anhydre porteuse de fragrance sans alcool comprenant :
au moins un composé parfumé, présent en une quantité d'environ 10 à 30 pour cent en poids par rapport à un poids total de la composition ;
du citrate de triéthyle, présent en une quantité d'environ 10 à 35 pour cent en poids par rapport au poids total de la composition ; et
de l'éthylhexylglycérine en une quantité d'environ 5 à 25 pour cent en poids par rapport au poids total de la composition.
